# EUROPEAN PATENT APPLICATION

(11) **EP 2 090 220 A1**
(43) Date of publication of application: **19.08.2009**
(21) Application number: 09001975.3
(22) Date of filing: 12.02.2009
(51) Int. Cl.: A61B 1/045

(54) **Processor for endoscope**

(30) Priority: 14.02.2008 JP 2008032730
(71) Applicant: FUJIFILM Corporation, Minato-ku Tokyo (JP)
(72) Inventor: Higuchi, Mitsuru, Saitama-shi, Saitama (JP)
(74) Representative: Höhfeld, Jochen

(57) **Abstract**

A processor (12) is provided with a power supply circuit (50) for supplying voltages of different values to a CCD (30). A RAM (47) of the processor stores a power control information table (60) in which types of the CCD and power control information indicating methods of supplying each voltage to the CCD are related and stored. A CPU (46) retrieves CCD information (34) indicating the type of the CCD from a ROM (32) and identifies the type of the CCD. The CPU then retrieves the power control information corresponding to the identified type from the power control information table. Based on the power control information, the CPU controls the power supply circuit to supply voltages of different values to the CCD in a sequence and at an interval corresponding to the type of the CCD.

## Description

### FIELD OF THE INVENTION

The present invention relates to a processor for endoscope that supplies electric power to an imaging sensor provided in the endoscope and also stores images from the endoscope.

### BACKGROUND OF THE INVENTION

An endoscope system includes an endoscope (scope) that takes images inside a body cavity and a processor that performs image processing to image data output from the endoscope and outputs the processed image data to a display device. The endoscope has an imaging sensor like a CCD, which is built in an end of an elongated insertion portion introduced into the body cavity. When the endoscope is provided with a power supply circuit for driving the CCD, the endoscope becomes large and heavy, which lowers operability of the endoscope. Endoscopy is sometimes performed a couple dozens times a day, and therefore reduction in size and weight of the endoscope is important in an attempt to reduce burden for operators of the endoscope. In view of this, the processor is often provided with the power supply circuit for driving the CCD in the general endoscope system, and electric power is supplied to the endoscope from the processor.

To drive the CCD, various voltages including positive voltage and negative voltage are necessary. A sequence and an interval for supplying each voltage are predetermined depending on the type of the CCD. When violating such driving procedure of the CCD, in the worst case, the CCD may be damaged. When the processor supplies each voltage according to the predetermined sequence and interval, only the endoscope with the corresponding CCD becomes available, which limits versatility of the processor.

In order to solve such problem, an endoscope disclosed in Japanese Patent Application Laid-open Publication No. 2003-38436 is provided with a CCD controller for controlling the supply of each voltage in a sequence and at an interval corresponding to the mounted CCD. When each voltage supply to the CCD is controlled at the endoscope side, the CCD can be prevented from damage, and the versatility of the processor will not be limited.

However, when the CCD controller is provided to the endoscope, same problem occurred in providing the power supply circuit for driving the CCD will arise. That is, the provision of the CCD controller to the endoscope requires enlargement of a circuit board, which hinders reduction in size and weight of the endoscope.

### SUMMARY OF THE INVENTION

An object of the present invention is to prevent damage of a CCD without limiting versatility of a processor and hindering reduction in size and weight of an endoscope.

In order to achieve the above and other objects, a processor for an endoscope according to the present invention includes a power supply circuit and a power control circuit. The power supply circuit supplies various voltages to an imaging sensor of an endoscope. The power control circuit controls the power supply circuit based on power control information including a sequence and an interval for supplying each of the voltages to the imaging sensor, and makes the power supply circuit supply each of the voltages in a sequence and at an interval corresponding to the power control information.

It is preferable that the power control circuit controls the power supply circuit to stop supplying each of the voltages in reverse sequence of starting drive of the imaging sensor when stopping the drive of the imaging sensor.

It is preferable that the endoscope has a memory for storing imaging sensor information indicating a type of the imaging sensor. In this case, the power control circuit retrieves the imaging sensor information from the memory. Based on the retrieved imaging sensor information, the power control circuit obtains the power control information corresponding to the imaging sensor of the connected endoscope by referring to table data in which the type of the imaging sensor and the power control information are related to each other.

It is also preferable that the endoscope has a memory for storing the power control information. In this case, the power control circuit obtains the power control information corresponding to the imaging sensor of the connected endoscope by retrieving the power control information stored in the memory.

It is preferable that a detecting section for detecting connection of the endoscope to the processor is further provided. After the connection of the endoscope is detected, the power control circuit controls the power supply circuit to supply electric power to the memory before supplying electric power to the imaging sensor.

It is preferable that a programmable device and a circuit forming section are further provided. In the programmable device, a drive circuit for driving the imaging sensor is formed. The programmable device is capable of rewriting the drive circuit. The circuit forming section forms the drive circuit corresponding to the imaging sensor in the programmable device.

Moreover, it is preferable that the power control information includes information regarding each of the voltages. The power supply circuit supplies each of the voltages corresponding to the information regarding each of the voltages.

According to the present invention, each of the voltages is supplied to the imaging sensor in the sequence and at the interval corresponding to the imaging sensor based on the power control information. Owing to this, the imaging sensor is prevented from damage when it is turned on. In addition, the present invention is applicable to various endoscopes with different types of imaging sensors by obtaining the power control information corresponding to the imaging sensor. Owing to this, the versatility of the processor is prevented from being limited. Moreover, since the supply of each voltage is controlled at the processor side, the present invention does not hinder reduction in size and weight of the endoscope.

### BRIEF DESCRIPTION OF THE DRAWINGS

One with ordinary skill in the art would easily understand the above-described objects and advantages of the present invention when the following detailed description is read with reference to the drawings attached hereto:
Figure 1 is a perspective view schematically illustrating a configuration of an endoscope system;
Figure 2 is a block diagram schematically illustrating configurations of an electronic endoscope and a processor;
Figure 3 is an explanatory view illustrating an example of a power control information table;
Figures 4A and 4B are explanatory views illustrating examples of a supply timing of each voltage;
Figure 5 is a flow chart illustrating an examination procedure of the endoscope system;
Figure 6 is a block diagram illustrating an example of storing power control information in a ROM of the electronic endoscope; and
Figure 7 is a block diagram illustrating an example of forming a CCD driver corresponding to a type of a CCD.

### DESCRIPTION OF THE PREFERRED EMBODIMENTS

Embodiments of the present invention are described hereinbelow. The present invention, however, is not limited to the following embodiments.

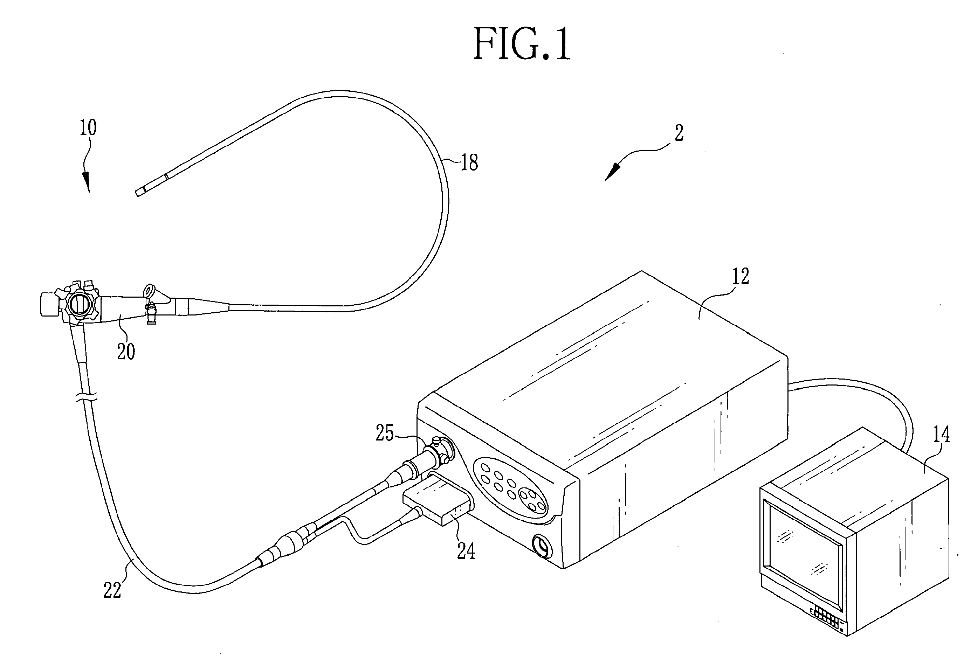

In Fig. 1, an endoscope system 2 includes an electronic endoscope 10 for taking images inside a body cavity of a patient, a processor 12 for producing endoscopic images, and a monitor 14 for displaying the endoscopic images. The electronic endoscope 10 is provided with an insertion portion 18 that is introduced into the body cavity, a handle 20 that is joined to a base end of the insertion portion 18, and a universal code 22 connected to the handle 20. The processor 12 is integrated with a light source for illuminating inside the body cavity. At the end of the universal code 22, opposite from the handle 20, a control connector 24 used for transmissions of electric power and various control signals and a light source connector 25 for taking in the illumination light emitted from a light source are provided. The electronic endoscope 10 is detachably connected to the processor 12 through the connectors 24 and 25.

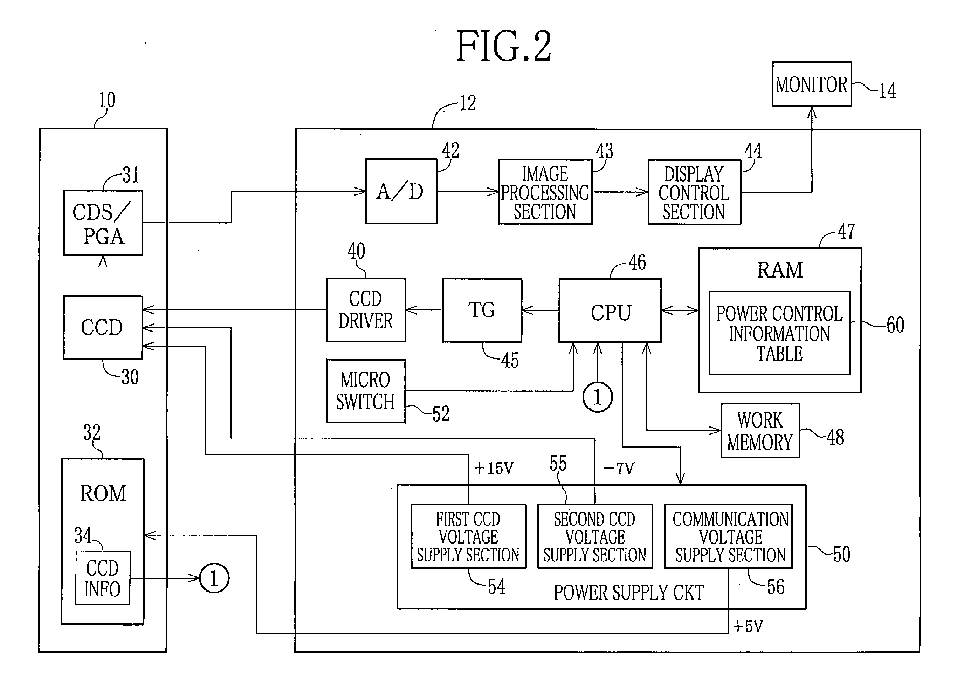

In Fig. 2, the electronic endoscope 10 is provided with a CCD (imaging sensor) 30, a correlation double sampling circuit/programmable gain amplifier (CDS/PGA) 31 and a ROM 32. The CCD 30 captures image light entering through an observation window formed at an end of the insertion portion 18. The CDS/PGA 31 performs denoising and amplification to the image signal output from the CCD 30. The ROM 32 stores CCD information (imaging sensor information) 34 indicating a type of the CCD 30. Note that the CCD information 34 may be a product name or a model number, or any other information as long as it identifies the type of the CCD 30.

The CCD 30 is connected to a CCD driver 40 provided to the processor 12. The CDS/PGA 31 is connected to an A/D converter 42 also provided to the processor 12.

The A/D converter 42 converts the analog image signal output from the CDS/PGA 31 to digital image data and outputs to an image processing section 43. The image processing section 43 performs various image processing to the image data digitized in the A/D converter 42. The processed image data is output to a display control section 44. The display control section 44 converts the image data output from the image processing section 43 to a video signal (for example, component signal, composite signal, or the like) in a form corresponding to the monitor 14 and outputs to the monitor 14. Thus, the endoscopic image obtained by taking inside the body cavity of the patient is displayed on the monitor 14.

The CCD driver 40 for driving the CCD 30 is connected to a timing generator (TG) 45. The TG 45 is connected to a CPU 46 that takes overall control of respective components of the processor 12. The TG 45 inputs, under the control of the CPU 46, a timing signal (clock pulse) to the CCD driver 40. The CCD driver 40 inputs a drive signal to the CCD 30 based on this timing signal, and thereby controlling the retrieving timing of the stored charge of the CCD 30, a shutter speed of electronic shutter of the CCD 30, and the like.

A RAM 47 storing various programs and data necessary for the control, a work memory 48 for temporarily storing information necessary for the control, a power supply circuit 50 for supplying electric power to the electronic endoscope 10, and a micro switch 52 for detecting a connection of the electronic endoscope 10 are connected to the CPU 46. The CPU 46 retrieves the programs stored in the RAM 47 and expands the programs to the work memory 48. The CPU 46 then sequentially process the retrieved programs, and thereby controlling the respective components of the processor 12. In addition, the CPU 46 is connected to the ROM 32 through the control connector 24 and the universal code 22.

The micro switch 52 is arranged such that its contact terminal is turned on when it is in contact with the control connector 24. The micro switch 52 detects the connection with the electronic endoscope 10 from the ON/OFF of the contact terminal and inputs the detection result to the CPU 46.

The power supply circuit 50 is provided with a first CCD voltage supply section 54, a second CCD voltage supply section 55, and a communication voltage supply section 56. The first CCD voltage supply section 54 is connected to the CCD 30 and supplies voltage of +15 V to the CCD 30. The second CCD voltage supply section 55 is also connected to the CCD 30 and supplies voltage of -7 V to the CCD 30. The communication voltage supply section 56 is connected to the ROM 32 and supplies voltage of +5 V to the ROM 32. The power supply circuit 50 controls start and stop of the electric power supply from each power supply section 54 to 56 according to the instruction from the CPU 46.

The power supply circuit 50 makes the first and second CCD voltage supply sections 54 and 55 supply different voltages to the CCD 30 and also makes the communication voltage supply section 56 supply the voltage to the ROM 32, and thereby activating the CCD 30 and the ROM 32. Owing to this, the image signal can be obtained from the CCD 30 and the CCD information 34 can be retrieved from the ROM 32.

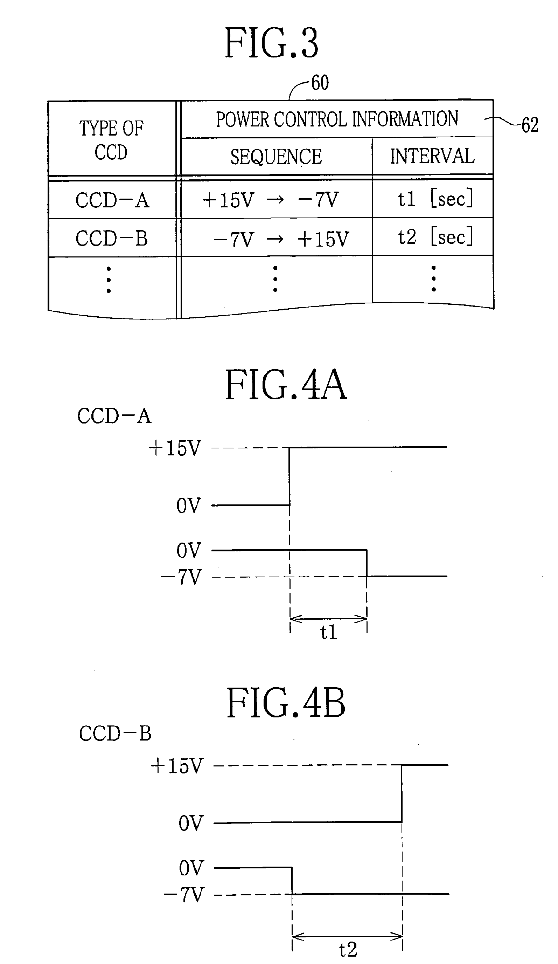

The RAM 47 stores a power control information table (table data) 60. The pattern (the sequence and the interval) for supplying the voltages differs depending on the type of the CCD 30. The power control information table 60 describes correspondence relations between the types of the CCD 30 and the patterns of supplying each of the voltages. As shown in Fig. 3, power control information 62 indicating the supply pattern of each voltage to the CCD 30 is recorded in relation with the type of the CCD 30. The power control information 62 includes the sequences of turning ON the first CCD voltage supply section 54 and the second CCD voltage supply section 55, and the interval to turn on one of the first and second CCD voltage supply sections 54 and 55 under the ON status of the other voltage supply section.

When the contact with the electronic endoscope 10 is detected by the micro switch 52, the CPU 46 instructs the power supply circuit 50 to supply the electric power to the ROM 32. Based on the instruction from the CPU 46, the power supply circuit 50 controls the communication voltage supply section 56 and supplies the electric power to the ROM 32. Thereafter, the CPU 46 accesses the ROM 32 and retrieves the CCD information 34 from the ROM 32. The CPU 46 stores the retrieved CCD information 34 in the work memory 48 and identifies the type of the CCD 30 based on the CCD information 34.

After the identification of the type of the CCD 30, the CPU 46 refers to the power control information table 60 and retrieves the power control information 62 corresponding to the identified type of the CCD 30. The CPU 46 then instructs the power supply circuit 50 to supply each of the voltages to the CCD 30 based on the retrieved power control information 62. The power supply circuit 50 controls the first CCD voltage supply section 54 and the second CCD voltage supply section 55 according to the instruction from the CPU 46 to supply each of the voltages to the CCD 30 in the sequence and at the interval corresponding to the power control information 62.

When the type of the CCD 30 is "CCD-A" as listed at the top of the power control information table 60, for example, the power supply circuit 50 firstly initiates the first CCD voltage supply section 54 to supply the voltage, as shown in Fig. 4A. t1 minutes later from the initiation of the voltage supply from the first CCD voltage supply section 54, the power supply circuit 50 initiates the second CCD voltage supply section 55 to supply the voltage. When the type of the CCD 30 is "CCD-B" as listed at the second top of the power control information table 60, the power supply circuit 50 firstly initiates the second CCD voltage supply section 55 to supply the voltage, as shown in Fig. 4B. t2 minutes later from the initiation of the voltage supply from the second CCD voltage supply section 55, the power supply circuit 50 initiates the first CCD voltage supply section 54 to supply the voltage.

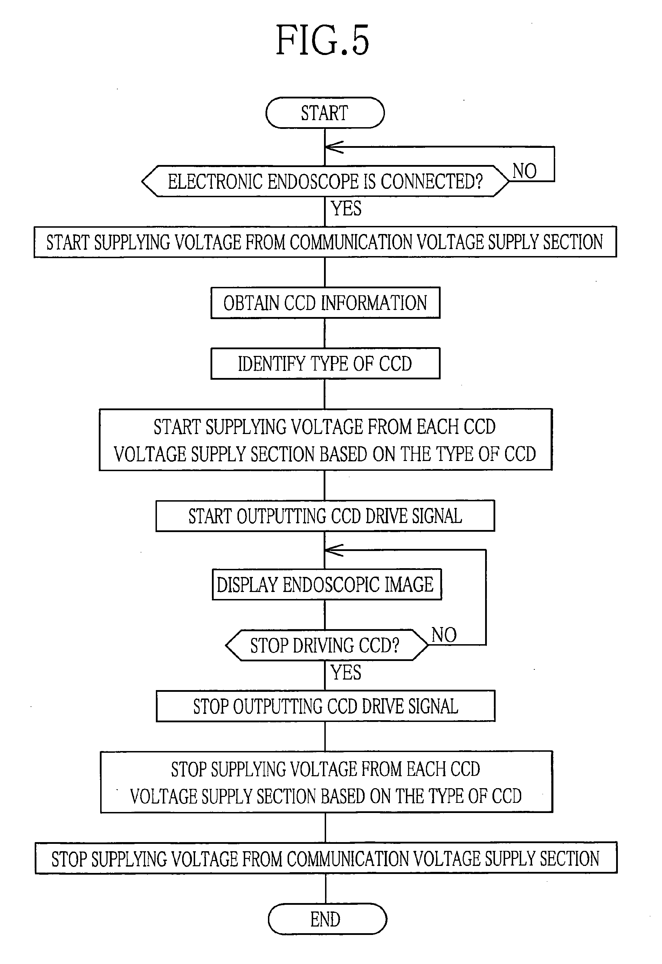

Now the operation of the endoscope system 2 having the above configuration will be described with reference to the flow chart shown in Fig. 5. To perform endoscopy using the endoscope system 2, the connectors 24 and 25 of the clean endoscope 10 which has been washed and sterilized are joined to the respective connectors of the processor 12, and thereby connecting the electronic endoscope 10 to the processor 12. When the electronic endoscope 10 is connected to the processor 12, the contact terminal of the micro switch 52 is turned on and the connection of the electronic endoscope 10 is detected.

When the connection of the electronic endoscope 10 is detected, the CPU 46 instructs the power supply circuit 50 to supply the electric power to the ROM 32 and retrieves the CCD information 34 from the ROM 32 as described above. The CPU 46 identifies the type of the CCD 30 of the connected electronic endoscope 10 based on the retrieved CCD information 34. After the identification of the type of the CCD 30, the CPU 46 refers to the power control information table 60 and retrieves the power control information 62 corresponding to the identified type of the CCD 30 from the power control information table 60. The CPU 46 then instructs the power supply circuit 50 to supply each of the voltages to the CCD 30 based on the retrieved power control information 62.

The power supply circuit 50 controls the first CCD voltage supply section 54 and the second CCD voltage supply section 55 according to the instruction from the CPU 46 to supply each of the voltages in the sequence and at the interval corresponding to the power control information 62. When the voltages are supplied in the sequence and at the interval corresponding to the type of the CCD 30 as described above, the CCD 30 is prevented from being damaged.

In addition, since the power control information 62 corresponding to the type of the CCD 30 is retrieved from the power control information table 60 based on the CCD information 34, the electronic endoscopes 10 incorporating various types of CCD 30 may be used with being connected to the processor 12. Therefore, the versatility of the processor 12 is prevented from limited. Moreover, since each voltage supply for the CCD 30 is controlled at the processor 12 side, the provision of the CCD controller to the electronic endoscope 10 can be omitted, which does not hinder reduction in size and weight of the electronic endoscope 10.

In the case where the electronic endoscope 10 is provided with the controller of the CCD 30 like the conventional endoscope, residual charge left in a capacitor which is provided between the CCD 30 and the controller may cause malfunction in the voltage supply sequence and interval when the electric power supply stops and restarts instantaneously due to instantaneous power failure. On the other hand, when the voltage supply from each voltage supply section to the CCD 30 is controlled by the processor 12, the residual charge left in the capacitor can instantly be lowered to ground, which enhances security against the instantaneous power failure as compared to the case where the controller is provided to the electronic endoscope 10.

After supplying the voltages to the CCD 30, the CPU 46 starts controlling the TG 45. The TG 45 inputs, under the control of the CPU 46, the timing signal to the CCD driver 40. The CCD driver 40 inputs the drive signal to the CCD 30 based on this timing signal, and thereby the image signal is output from the CCD 30 and the endoscopic image is displayed on the monitor 14.

When it is instructed to stop driving the CCD 30 by the user after the endoscopy, the CPU 46 makes the CCD driver 40 stop inputting the drive signal to the CCD 30 and also instructs the power supply circuit 50 to stop supplying the electric power to the CCD 30. Based on the instruction from the CPU 46, the power supply circuit 50 controls the first CCD voltage supply section 54 and the second voltage supply section 55 to stop the voltage supply in reverse sequence of turning them on. Since the drive of the CCD 30 is stopped in reverse sequence of the turning-on sequence, the CCD 30 is prevented from damage at the time of stopping the drive thereof.

After stopping the supply of the electric power to the CCD 30, the CPU 46 instructs the power supply circuit 50 to stop supplying the electric power to the ROM 32. According to the instruction from the CPU 46, the power supply circuit 50 controls the communication voltage supply section 56 to stop the voltage supply to the ROM 32. The electronic endoscope 10 can be safely removed from the processor 12 by stopping the electric power supply to the CCD 30 and the ROM 32 in the sequence as described above.

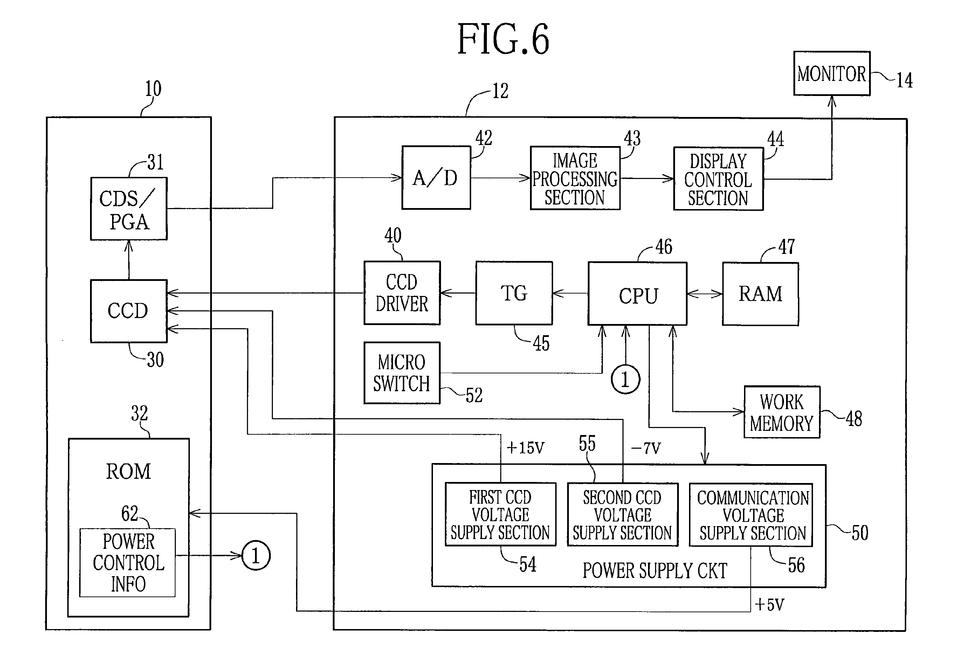

In the above embodiment, the CCD information 34 is retrieved from the ROM 32 provided in the electronic endoscope 10 and the power control information 62 is obtained by referring to the power control information table 60 based on the CCD information 34. However, the method for obtaining the power control information 62 is not limited to this. For example, as shown in Fig. 6, the power control information 62 corresponding to the CCD 30 incorporated in the electronic endoscope 10 may be stored in the ROM 32, and the power control information 62 can be obtained by retrieving from the ROM 32. In this case, the power control information table 60 need not be stored in the RAM 47. Moreover, the power control information 62 may be manually input. In this case, the ROM 32 is not needed either.

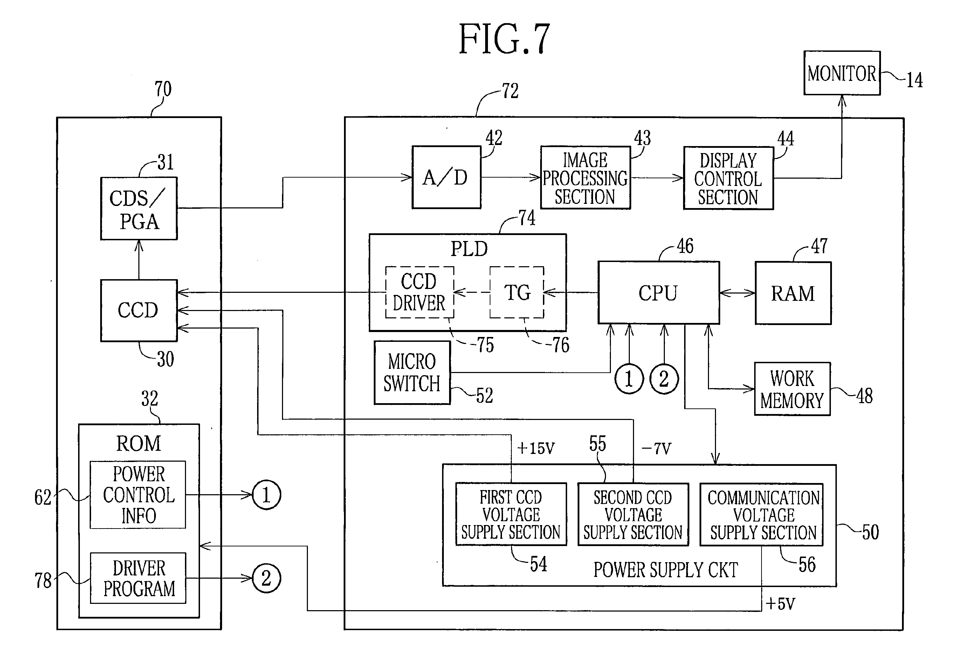

In the above embodiment, only one CCD driver 40 and one TG 45 are provided to operate different types of the CCD 30. However, the drive signal or the clock frequency may vary depending on the type of the CCD 30. When clock pulses for the drive signal differs between the CCD driver 40 or the TG 45 and the CCD 30, the CCD 30 cannot be operated. To solve such problem, an electronic endoscope 70 and a processor 72 as shown in Fig. 7 may be used.

The processor 72 is provided with a programmable logic device (PLD) 74 that can rewrite a logic circuit. The ROM 32 of the electronic endoscope 70 stores a driver program 78 for forming a CCD driver 75 and a TG 76 corresponding to the CCD 30 of the electronic endoscope 70 in the PLD 74.

When the connection of the electronic endoscope 70 is detected, the CPU 46 instructs the power supply circuit 50 to supply the electric power to the ROM 32 and retrieves the power control information 62 and the driver program 78 from the ROM 32. The CPU 46 instructs the power supply circuit 50 to supply each of the voltages to the CCD 30 based on the retrieved power control information 62, in the sequence and at the interval corresponding to the power control information 62.

Upon supplying electric power to the CCD 30, the CPU 46 also writes the retrieved driver program 78 to the PLD 74 and forms the CCD driver 75 and the TG 76 that correspond to the CCD 30 of the electronic endoscope 70 in the PLD 74. That is, the CPU 46 works as the circuit forming section. The formation of the CCD driver 75 and the TG 76 corresponding to the CCD 30 in the PLD 74 surely prevents the problem of not being able to operate the CCD 30 due to difference of the clock pulses for the drive signal. In Fig. 7, although the PLD 74 is shown as the programmable device, a field programmable gate array (FPGA) for example may also be used.

In the above embodiment, the first CCD voltage supply section 54 supplies voltage of +15 V and the second CCD voltage supply section 55 supplies voltage of -7 V, respectively to the CCD 30. However, the drive voltage of the CCD 30 may vary depending on the type of the CCD 30. To supply the voltage corresponding to the type of the CCD 30 from the power supply circuit 50, for example, DC/DC converters with different voltages may be provided and the converter may be selectively switched depending on the type of the CCD 30. It is also possible to perform resistive division of the voltage using an electronic volume circuit. In supplying the voltage corresponding to the type of the CCD 30, the voltage value may be included in the power control information 62 in advance, so that the corresponding voltage value can be identified by referring to the power control information 62.

In the above embodiment, although the ROM 32 is shown as the memory, other memory devices such as for example a RFID tag may be used as long as it can store the CCD information 34 and the power control information 62. In addition, in the above embodiment, although the CCD 30 is shown as the imaging sensor, for example, a CMOS imaging sensor may also be used. Moreover, in the present invention, for example, an optical sensor like a photo interrupter or a magnetic sensor like a hall element may be used instead of the micro switch 52.

Note that the endoscope may be, for example, an ultrasonic endoscope besides the electronic endoscope 10. The present invention may also be applied to an endoscope used for industrial purposes for observing through pipes and the like. Furthermore, instead of the processor 12 that is integrated with the light source, the present invention may be applied to a processor provided separately from a light source.

Various changes and modifications are possible in the present invention and may be understood to be within the present invention.

## Claims

1. A processor (12) for an endoscope (10) to which said endoscope including an imaging sensor (30) is detachably connected, said processor comprising:
a power supply circuit (50) for supplying various voltages to said imaging sensor; and
a power control circuit (46) for controlling said power supply circuit based on power control information (62) including a sequence and an interval for supplying each of said voltages to said imaging sensor, said power control circuit making said power supply circuit supply each of said voltages in a sequence and at an interval corresponding to said power control information.

2. The processor described in claim 1, wherein said power control circuit controls said power supply circuit to stop supplying each of said voltages in reverse sequence of starting drive of said imaging sensor when stopping said drive of said imaging sensor.

3. The processor described in claim 1, wherein said endoscope has a memory (32) for storing imaging sensor information (34) indicating a type of said imaging sensor, and wherein
said power control circuit retrieves said imaging sensor information from said memory,
based on said retrieved imaging sensor information, said power control circuit obtaining said power control information corresponding to said imaging sensor of the connected endoscope by referring to table data (60) in which said type of said imaging sensor and said power control information are related to each other.

4. The processor described in claim 1, wherein said endoscope has a memory (32) for storing said power control information, and wherein
said power control circuit obtains said power control information corresponding to said imaging sensor of the connected endoscope by retrieving said power control information stored in said memory.

5. The processor described in claim 4, further including:
a detecting section (52) for detecting connection of said endoscope to said processor, and wherein
after the connection of said endoscope is detected, said power control circuit controls said power supply circuit to supply electric power to said memory before supplying electric power to said imaging sensor.

6. The processor (70) described in claim 1, further including:
a programmable device (74) in which a drive circuit (75) for driving said imaging sensor is formed, said programmable device being capable of rewriting said drive circuit; and
a circuit forming section (78) for forming said drive circuit corresponding to said imaging sensor in said programmable device.

7. The processor described in claim 6, wherein said power control information includes information regarding each of said voltages and said power supply circuit supplies each of said voltages corresponding to said information regarding each of said voltages.
